# EUROPEAN PATENT SPECIFICATION

(11) **EP 0 786 255 B1**
(45) Date of publication and mention of the grant of the patent: **12.12.2001**
(21) Application number: 96117245.9
(22) Date of filing: 06.10.1992
(51) Int. Cl.: A61K 38/17

(54) **Methods of improving allograft or xenograft tolerance by administration of an LFA-3 or CD2 binding protein**
Verfahren zur Verbesserung der Toleranz für Allotransplantaten und Xenotransplantaten durch Verabreichung eines LFA-3- oder CD2-Bindungsproteins
Procédés d'amélioration de la tolérance des greffes allogènes ou xénogènes par administration d'une protéine liante à LFA-3 ou CD2

(30) Priority: 07.10.1991 US 772705; 12.03.1992 US 850706
(43) Date of publication of application: 30.07.1997
(62) Divisional of application: 92922682.7
(73) Proprietor: BIOGEN, INC., Cambridge Massachusetts 02142 (US)
(72) Inventor: Wallner, Barbara, Cambridge, MA 02139 (US); Benjamin, Christopher, Beverly, MA 01915 (US)
(74) Representative: Ruffles, Graham Keith

(56) References cited:
- EP-A- 0 280 578
- EP-A- 0 503 648
- WO-A-88/09820
- WO-A-90/02181
- WO-A-91/11194
- THE JOURNAL OF EXPERIMENTAL MEDICINE, vol. 166, no. 4, 1 October 1987, NEW YORK, NY, USA, pages 923-932, XP000673505 B. WALLNER ET AL.: "Primary structure of lymphocyte function-associated antigen 3 (LFA-3)."

## Description

### TECHNICAL FIELD OF INVENTION

The present invention relates to methods of improving tolerance of transplanted xenograft tissue or allograft tissue by administration of LFA-3 or CD2 binding proteins in mammals, including humans.

### BACKGROUND OF THE INVENTION

An allograft is tissue that is transplanted between genetically nonidentical members of the same species. Allografts of organs such as the heart, kidney, liver, pancreas, cornea, bone marrow, lung and skin have become an increasingly successful and accepted medical practice for the treatment of various end stage diseases. The resulting increase in demand for transplants, unfortunately, has not been matched by an increase in the present donor supply, and efforts to increase the supply of human donors are not predicted to match the rising demand for human organs. For example, only 2,000 of the 14,000 patients per year who are eligible for a cardiac allograft actually receive a heart transplant in the United States (Rose, "Risks Of Cardiac Transplantation", Ann. Thorac. Surg., 47, p. 615 (1989)).

Consequently, interest has increased in alternative sources for donor organs. One such alternative source is xenografts, which are transplants of tissue from one species to another species.

A problem for both allografts and xenografts is rejection of the donor graft tissue by the recipient. Graft rejection is the result of a complicated and not fully understood chain of events in the immune system. There are generally two facets of the immune response: 1) a cell mediated response, primarily comprising cytotoxic T cells which attack and kill foreign cells or virus-infected cells; and 2) a humoral response, comprising the activation of B cells to plasma cells which secrete antibodies specific for foreign macromolecules.

Graft rejection is histologically characterized by the progressive infiltration of mononuclear cells, including lymphocytes, into the foreign tissue. The increased presence of these cells precedes the destruction of the graft by several days. Sensitized T lymphocytes, therefore, appear to be the principal initiators of the rejection process.

T lymphocytes play a major role in the immune response by interacting with target and antigen-presenting cells. For example, T lymphocyte-mediated killing of target cells is a multi-step process involving, initially, adhesion of cytolytic T lymphocytes (the effector cells) to target cells, such as graft endothelium. Also, helper T lymphocytes help initiate the immune response by adhesion to antigen-presenting cells within the graft tissue.

These interactions of T lymphocytes with target and antigen-presenting cells are highly specific and depend on the recognition of an antigen on the surface of a target or antigen-presenting cell by one of the many specific antigen receptors on the surface of T lymphocytes.

The receptor-antigen interaction of T lymphocytes and other cells is also facilitated by various T lymphocyte surface proteins, e.g., the antigen-receptor complex CD3 and accessory molecules such as CD4, LFA-1, CD8, and CD2. It is also affected by accessory molecules such as LFA-3, ICAM-1 and MHC that are expressed on the surface of the target or antigen-presenting cells.

The interaction between CD2 and LFA-3 remains poorly understood with respect to activation of T cell activity. Recent studies have suggested that there is a specific interaction between CD2 (a T lymphocyte accessory adhesion molecule) and LFA-3 (a target cell and antigen presenting cell accessory molecule) which mediates T lymphocyte adhesion to the target or antigen presenting cell. This cell-cell adhesion has been implicated in the initiation of T lymphocyte functional responses (Dustin et al., "Purified Lymphocyte Function Associated Antigen 3 Binds To CD2 And Mediates T lymphocyte Adhesion," J. Exp. Med., 165, pp. 677-92 (1987); Springer et al., "The Lymphocyte Function-associated LFA-1, CD2, and LFA-3 Molecules: Cell Adhesion Receptors of the Immune System", Ann. Rev. Immunol., 5, pp. 223-52 (1987)). The LFA-3/CD2 interaction also plays a role in mediating T lymphocyte interactions with thymic epithelial cells, in antigen-independent and dependent conjugate formation and in T lymphocyte rosetting with erythrocytes (see, e.g., Seed et al., "Molecular Cloning Of The CD2 Antigen, the T-Cell Erythrocyte Receptor, By a Rapid Immunoselection Procedure", Proc. Natl. Acad. Sci. USA, 84, pp. 3365-69 (1987)).

LFA-3, which is found on the surface of a wide variety of cells, including human erythrocytes, has become the subject of a considerable amount of study to further elucidate its role in various T lymphocyte interactions (see, e.g., Krensky et al., "The Functional Significance, Distribution, and Structure of LFA-1, LFA-2, and LFA-3: Cell Surface Antigen Associated with CTL-Target Interactions", J. Immunol., 131(2), pp. 611-16 (1983); Shaw et al., "Two Antigen-Independent Adhesion Pathways Used by Human Cytotoxic T-cell Clones", Nature, 323, pp. 262-64 (1986)). Two natural forms of LFA-3 have been identified. One form of LFA-3 ("transmembrane LFA-3") is anchored in the cell membrane by a transmembrane hydrophobic domain. cDNA encoding this form of LFA-3 has been cloned and sequenced (see, e.g., Wallner et al., "Primary Structure of Lymphocyte Function-Associated Antigen-3 (LFA-3)", J. Exp. Med., 166, pp. 923-32 (1987)). Another form of LFA-3 is anchored to the cell membrane via a covalent linkage to phosphatidylinositol ("PI")-containing glycolipid. This latter form has been designated "PI-linked LFA-3", and cDNA encoding this form of LFA-3 has also been cloned and sequenced (Wallner et al., PCT publn. WO 90/02181).

The human CD2 (T11) molecule is a 50 kD surface glycoprotein expressed on >95% of thymocytes and virtually all peripheral T lymphocytes. Biochemical analyses using specific monoclonal antibodies have suggested that CD2 is T lineage-specific and exists on the cell surface in several differentially glycosylated forms (Howard et al., "A Human T Lymphocyte Differentiation Marker Defined by Monoclonal Antibodies that Block E-Rosette Formation", J. Immunol., 126, pp. 2117-22 (1981); Brown et al., in Leukocyte Typing III, ed. McMichael, Oxford University Press, pp. 110-12 (1987); Sayre et al., "Molecular Cloning and Expression of T11 cDNAs Reveals a Receptor-Like Structure on Human T lymphocytes", Proc, Natl, Acad, Sci, USA, 84, pp. 2941-45 (1987)). The sequence of a human CD2 gene has been reported (Seed and Aruffo, "Molecular Cloning of the CD2 Antigen, the T-cell Erythrocyte Receptor, by a Rapid Immunoselection Procedure", Proc. Natl. Acad. Sci. USA, 84, pp. 3365-69 (1987); Sayre et al., supra (1987). Soluble CD2 polypeptides having an LFA-3 binding domain have been reported (PCT publ. WO 90/08187).

Monoclonal antibodies to CD2, e.g, TS2/18, T11₁, T11₂, T11₃, and to LFA-3, e.g., TS2/9, have also been reported (see, e.g., Hughes et al., "The Endothelial Cell as a Regulator of T-Cell Function", Immunol. Reviews, 117, pp. 85-102 (1990); Meuer, "An Alternative Pathway of T-Cell Activation: A Functional Role for the 50 kd T11 Sheep Erythrocyte Receptor Protein", Cell, 36, pp. 897-906 (1984); Sanchez-Madrid et al., "Three Distinct Antigens Associated with Human T-Lymphocyte-Mediated Cytolysis: LFA-1, LFA-2, and LFA-3", Proc. Natl. Acad. Sci. USA, 79, pp. 7489-93 (1982)); Bromberg et al., Transplantation, 51, pp. 219-225 (1991); EP 0 260 880 A2.

Suppression of the immune response to prevent graft rejection has previously been effected by drugs, such as prednisone, cyclosporine, azathioprine or cyclophosphamide, which nonspecifically block cell-mediated responses. Irradiation has also been used to destroy T and B lymphocytes that could react against the transplanted graft tissue. Immunosuppression with the above techniques, however, cannot produce antigen-specific tolerance and, therefore, greatly increases the patient's susceptibility to opportunistic infection. In addition, other detrimental side effects will occur with chronic use of the above immunosuppression techniques, For example, chronic cyclosporine treatment is associated with a high incidence of renal toxicity, hypertension and malignant neoplasm.

Cytotoxic T lymphocyte mediated responses are controlled by cyclosporine or prednisone, but immune suppressive therapy is ineffectual for humoral rejection episodes. Currently, there is no therapeutic intervention for humoral rejection.

To date, therefore, conventional methods and therapeutic agents have not proved to be satisfactory for improving tolerance of xenografts or allografts. Accordingly, the need still exists for a process which avoids the disadvantages of the conventional methods and agents while providing an effective method for decreasing the severity of rejection of graft tissue.

### SUMMARY OF THE INVENTION

The present invention generally solves many of the problems referred to above. It, for the first time, provides the use of a protein that binds CD2, which is a derivative of a soluble LFA-3 polypeptide, the derivative being an immunoglobulin fusion comprising the soluble LFA-3 polypeptide fused to an immunoglobulin region in the preparation of a medicament for use in a method of improving tolerance of transplanted allograft tissue or xenograft tissue in a mammal, including a human, wherein the method comprises implanting in the mammal an allograft or a xenograft and administering the protein. The methods of the invention will preferably be used to improve tolerance of cardiac and renal xenografts and to previously available therapies for improving graft tolerance for many reasons, including avoidance of undesirable side effects such as increased susceptibility to opportunistic infection, renal toxicity, hypertension and malignant neoplasm.

### BRIEF DESCRIPTION OF THE DRAWINGS

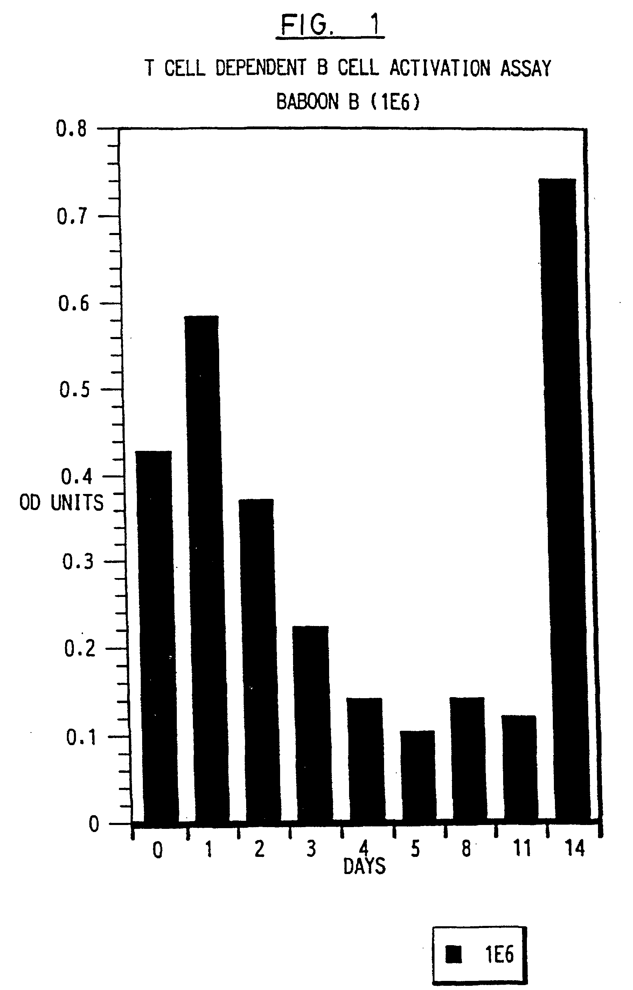

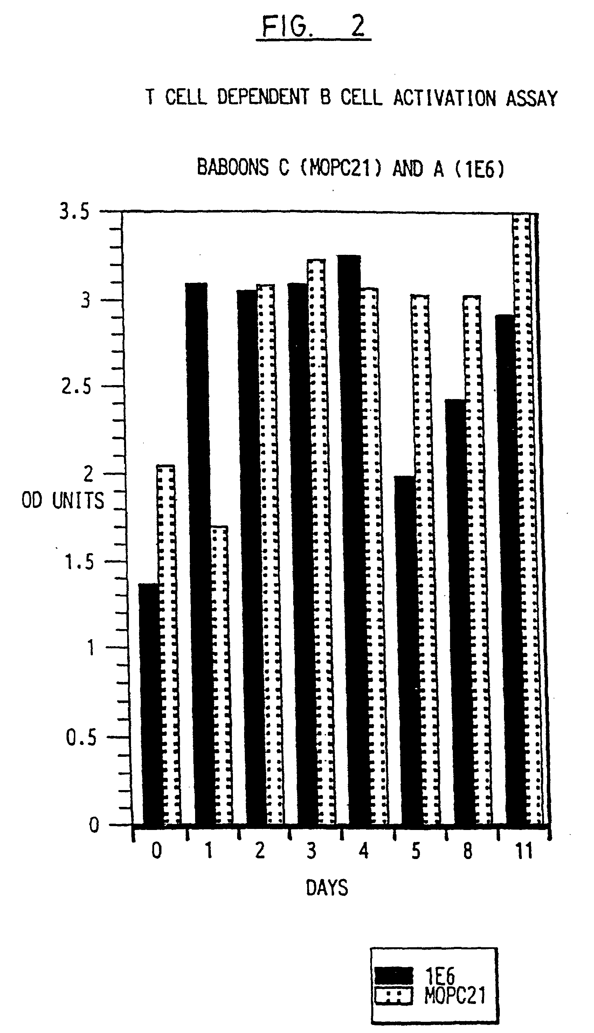

Figures 1 and 2 illustrate T cell dependent B cell activation assay results for two baboons injected with an anti-LFA-3 monoclonal antibody (1E6) and one baboon injected with a non-specific isotype matched control monoclonal antibody (MOPC21). Immunoglobulin production as measured by OD units in an ELISA assay is reflected on the y axes. The number of days after the initial injection of anti-LFA-3 monoclonal antibody is illustrated on the x axes.

### DETAILED DESCRIPTION OF THE INVENTION

### Definitions

As used herein, an "LFA-3 binding protein" is a protein comprising one or more polypeptides capable of binding to LFA-3. LFA-3 binding proteins include immunoglobulin light chains, immunoglobulin heavy chains and antigen-binding fragments thereof. The component polypeptides of an LFA-3 binding protein composed of more than one polypeptide may optionally be disulfide-bound or otherwise covalently crosslinked. Accordingly, LFA-3 binding proteins include intact immunoglobulins of types IgA, IgG, IgE, IgD, IgM (as well as subtypes thereof), wherein the light chains of the immunoglobulin may be of types kappa or lambda. Such binding proteins also include portions of intact immunoglobulins that retain LFA-3-binding specificity, for example, Fab fragments, Fab' fragments, F(ab')₂ fragments, F(v) fragments, heavy chain monomers or dimers, light chain monomers or dimers, dimers consisting of one heavy and one light chain, and the like.

Also contemplated within the term "LFA-3 binding protein" are soluble CD2 polypeptides and derivatives thereof, including fusions, that bind to LFA-3. As used herein, a "soluble CD2 polypeptide" is a CD2 polypeptide incapable of anchoring itself in a cell membrane. Such soluble polypeptides include, for example, CD2 polypeptides that lack a sufficient portion of their membrane-spanning domain to anchor the polypeptide or are modified such that the membrane-spanning domain is nonfunctional. Soluble CD2 polypeptides bind to a naturally occurring LFA-3 polypeptide and are encoded by (a) a naturally occurring mammalian CD2 DNA sequence (e.g., SEQ ID NO:5), (b) a DNA sequence degenerate to a naturally occurring CD2 DNA sequence or (c) a DNA sequence that hybridizes to one of the foregoing DNA sequences under conditions equivalent to about 20°C to 27°C below Tₘ and 1 M sodium chloride. Such soluble CD2 polypeptides are well known. For example, several are described in PCT WO 90/08187, which is herein incorporated by reference.

As used herein, a "CD2 binding protein" is a protein comprising one or more polypeptides capable of binding to CD2. CD2 binding proteins include immunoglobulin light chains, immunoglobulin heavy chains and antigen-binding fragments thereof. The component polypeptides of a CD2 binding protein composed of more than one polypeptide may optionally be disulfide-bound or otherwise covalently crosslinked. Accordingly, CD2 binding proteins include intact immunoglobulins of types IgA, IgG, IgE, IgD, IgM (as well as subtypes thereof), wherein the light chains of the immunoglobulin may be of types kappa or lambda. Such binding proteins also include portions of intact immunoglobulins that retain CD2-binding specificity, for example, Fab fragments, Fab' fragments, F(ab')2 fragments, F(v) fragments, heavy chain monomers or dimers, light chain monomers or dimers, dimers consisting of one heavy and one light chain, and the like.

Also contemplated within the term "CD2 binding protein" are soluble LFA-3 polypeptides or derivatives thereof, including fusions, that bind to CD2. As defined herein, CD2 binding proteins include fusions of soluble LFA-3 polypeptides and immunoglobulin regions, such as LFA3TIP (described infra). As used herein, a "soluble LFA-3 polypeptide" is a LFA-3 polypeptide incapable of anchoring itself in a cell membrane. Such soluble polypeptides include, for example, LFA-3 polypeptides that lack a sufficient portion of their membrane-spanning domain to anchor the polypeptide or are modified such that the membrane-spanning domain is nonfunctional. Soluble LFA-3 polypeptides bind to a naturally occurring CD2 polypeptide and are encoded by (a) a naturally occurring mammalian LFA-3 DNA sequence (e.g. SEQ ID NO:1 or SEQ ID NO:3, (b) a DNA sequence degenerate to a naturally occurring LFA-3 DNA sequence or (c) a DNA sequence that hybridizes to one of the foregoing DNA sequences under conditions equivalent to about 20°C to 27°C below Tₘ and 1 M sodium chloride. Such soluble LFA-3 polypeptides are well known. For example, several are described in United States patent 4,956,281, which is herein incorporated by reference.

As used herein, a "humanized recombinant antibody" is an antibody, produced by recombinant DNA technology, in which some or all of the amino acids of a human immunoglobulin light or heavy chain not required for antigen binding have been substituted for the corresponding amino acids from a nonhuman mammalian immunoglobulin light or heavy chain.

As used herein, a "chimeric recombinant antibody" is an antibody produced by recombinant DNA technology, in which all or part of the hinge and constant regions of an immunoglobulin light chain, heavy chain or both, have been substituted for the corresponding regions from another immunoglobulin light chain or heavy chain.

As used herein, "improving tolerance" of transplanted graft tissue is decreasing the severity of or eliminating one or more of the general characteristics of graft rejection. Such characteristics evidence immune response directed against the graft (foreign) tissue and include, for example, progressive infiltration of mononuclear cells, such as lymphocytes, into the foreign tissue, production of lymphocytotoxic antibodies, cytolysis, necrosis, vasculitis, hemorrhage and fibrosis. Another readily observable indication of improved tolerance will be prolonged survival of transplanted graft tissue in a recipient as compared to a non-immunosuppressed recipient (control).

### Graft Tissue

The present invention is useful in methods of improving tolerance in mammals, including humans, of transplanted allograft tissue or xenograft tissue. They comprise the steps of administering to the mammal a graft tissue and a CD2 binding protein. Such grafts include allografts and xenografts of tissues derived from sources including the heart, kidney, liver, pancreas, cornea, bone marrow, lung, skin and blood. Such tissues include portions of the organs mentioned above and subfractions of blood. Preferably, the invention is used for cardiac allografts and xenografts, and renal allografts and xenografts. The methods of the invention can be practiced on any mammal, preferably humans.

In selecting graft tissue, a variety of factors should be considered. These include, for example, a minimization of genetic disparity to the extent possible, ABO blood group compatibility, HLA compatibility, the availability of donor tissue, the immune status of the patient and size of the donor organ. Specifically, in the case of cardiac and renal allografts or xenografts, the donor organ should be anatomically compatible and physiologically competent to support the organ function requirements of the recipient. Surgical protocols used for various graft transplants are well known.

While not wishing to be bound by theory, applicants believe that the CD2 binding proteins used in the methods of this invention are prophylactic and therapeutic for inducing tolerance of the xenografts or allografts because they inhibit T cell activation. This inhibition typically occurs when the CD2 binding protein inhibits the LFA-3/CD2 interaction. However, certain CD2 binding proteins used in this invention may inhibit T cell activation without inhibiting the LFA-3/CD2 interaction.

Preferred CD2 binding proteins for use in the methods of this invention are effective to inhibit T cell activation.

The utility in the methods of this invention of specific CD2 binding proteins may easily be determined by assaying their ability to inhibit the LFA-3/CD2 interaction, their ability to inhibit T cell activation or both.

The ability to inhibit the LFA-3/CD2 interaction may be assayed, for example, using a simple cell binding assay that permits visual (under magnification) evaluation of the ability of the putative inhibitor to inhibit the interaction between LFA-3 and CD2 on cells expressing these molecules. Jurkat cells are preferred as the CD2⁺ substrate and sheep red blood cells or human JY cells are preferred as the LFA-3⁺ substrate. The binding characteristics of binding proteins useful in this invention may be assayed in several known ways, such as by radiolabeling the binding protein (e.g., with ³⁵S or ¹²⁵I) and then contacting the labeled binding protein with CD2⁺ or LFA-3⁺ cells, as appropriate. Binding characteristics may also be assayed using an appropriate enzymatically labelled secondary antibody. Rosetting competition assays, such as those described in Seed et al., Proc. Natl. Acad. Sci. USA, 84, pp. 3365-69 (1987) may also be used.

The ability of CD2 binding proteins to inhibit T cell activation may be determined in any number of conventional T cell activation assays. These include, for example, assays which assess the ability of the binding protein to inhibit T cell proliferation or cytokine secretion in response to mitogens or activating monoclonal antibodies directed to other cell surface proteins (see, e.g., Moingeon et al., "The Structural Biology of CD2", Immunological Rev., 111, pp. 111-44 (1989)).

### CD2 Binding Proteins

Many types of CD2 binding proteins are useful in this invention, including, soluble LFA-3 polypeptides, as well as derivatized (e.g., fused to another polypeptide) or truncated forms of any of the foregoing.

### B. Soluble LFA-3 Polypeptides

The CD2 binding proteins useful in the methods of the present invention include soluble LFA-3 polypeptides, which are preferred.

Soluble LFA-3 polypeptides may be derived from the transmembrane form of LFA-3, particularly the extracellular domain (e.g., AA₁-AA₁₈₇ of SEQ ID NO:2). Such polypeptides are described in United States patent 4,956,281 and US 554 7853. Preferred soluble LFA-3 polypeptides include polypeptides consisting of AA₁-AA₉₂ of SEQ ID NO:2, AA₁-AA₈₀ of SEQ ID NO:2, AA₅₀-AA₆₅ of SEQ ID NO:2 and AA₂₀-AA₈₀ of SEQ ID NO:2. A bacteriophage comprising a DNA sequence encoding SEQ ID NO:2 (i.e., SEQ ID NO:1) is deposited with American Type Culture Collection, Rockville, Maryland, under the accession number ATCC 75107.

Soluble LFA-3 polypeptides may also be derived from the PI-linked form of LFA-3, such as those described in PCT patent application WO 90/02181. A vector comprising a DNA sequence encoding PI-linked LFA-3 (i.e., SEQ ID NO:3) is deposited with American Type Culture Collection, Rockville, Maryland, under the accession number ATCC 68788. Since the PI-linked form of LFA-3 and the transmembrane form of LFA-3 have identical amino acid sequences through the entire extracellular domain, the preferred soluble LFA-3 polypeptides derived from PI-linked LFA-3 are the same as those derived from the transmembrane form of LFA-3.

The production of the soluble polypeptides useful in this invention may be achieved by a variety of methods known in the art. For example, the polypeptides may be derived from intact transmembrane LFA-3 molecules or an intact PI-linked LFA-3 molecule by proteolysis using specific endopeptidases in combination with exopeptidases, Edman degradation, or both. The intact LFA-3 molecule, in turn, may be purified from its natural source using conventional methods. Alternatively, the intact LFA-3 may be produced by known recombinant DNA techniques using cDNAs (see, e.g., U.S. Patent 4,956,281 to Wallner et al.; Aruffo and Seed, Proc. Natl. Acad. Sci. USA, 84, pp. 2941-45 (1987); Sayre et al., Proc. Natl. Acad. Sci. USA, 84, pp. 2941-45 (1987)).

Preferably, the soluble polypeptides useful in the present invention are produced directly, thus eliminating the need for obtaining an entire LFA-3 molecule as a starting material. This may be achieved by conventional chemical synthesis techniques or by well-known recombinant DNA techniques wherein only those DNA sequences which encode the desired polypeptides are expressed in transformed hosts. For example, a DNA sequence which encodes the desired soluble LFA-3 polypeptide may be synthesized by chemical means using an oligonucleotide synthesizer. Such oligonucleotides are designed based on the amino acid sequence of the desired soluble LFA-3 polypeptide. Specific DNA sequences coding for the desired polypeptide also can be derived from the full length DNA sequence by isolation of specific restriction endonuclease fragments or by PCR synthesis of the desired region.

The soluble LFA-3 polypeptides may be isolated from the fermentation or culture of transfected host cells and purified using any of a variety of conventional methods. One of skill in the art may select the most appropriate isolation and purification techniques.

While recombinant DNA techniques are the preferred method of producing useful soluble LFA-3 polypeptides having a sequence of more than 20 amino acids, shorter LFA-3 polypeptides having less than about 20 amino acids are preferably produced by conventional chemical synthesis techniques. Synthetically produced polypeptides useful in this invention can advantageously be produced in extremely high yields and can be easily purified.

### D. Derivatized CD2 Binding Proteins

Preferred derivatized binding proteins include recombinantly produced polypeptides in which a soluble LFA-3 polypeptide, is fused to all or part of an immunoglobulin heavy chain hinge region and all or part of an immunoglobulin heavy chain constant region. Such fusion proteins are expected to exhibit prolonged serum half-lives and to facilitate binding protein dimerization.

Preferred polypeptides for preparing such fusion proteins are soluble LFA-3 polypeptides, most preferably a soluble LFA-3 polypeptide selected from the group consisting of AA₁-AA₉₂ of SEQ ID NO:2, AA₁-AA₈₀ of SEQ ID NO:2, AA₅₀-AA₆₅ of SEQ ID NO:2 and AA₂₀-AA₈₀ of SEQ ID NO:2.

A bacteriophage comprising a DNA sequence encoding SEQ ID NO:2 (i.e., SEQ ID NO:1) is deposited with the American Type Culture Collection, Rockville, Maryland, under the accession number ATCC 75107.

The most preferred fusion proteins of this type contain the amino terminal 92 amino acids of mature LFA-3, the C-terminal 10 amino acids of a human IgG₁ hinge region containing the two cysteine residues thought to participate in interchain disulfide bonding, and the C_{H}2 and C_{H}3 regions of a human IgG₁ heavy chain constant domain (e.g., SEQ ID NO:8). This fusion protein is referred to herein as "LFA3TIP." A plasmid, pSAB152, encoding an exemplary LFA3TIP is deposited with American Type Culture Collection, Rockville, Maryland, under the accession number ATCC 68720. The DNA sequence of the pSAB152 insert is SEQ ID NO:7.

One way of producing LFA3TIP for use in the methods of this invention is described in co-pending, commonly assigned United States patent application 07/770,967. Generally, conditioned culture medium of COS7 cells transfected with pSAB152 was concentrated using an AMICON S1Y30 spiral cartridge system (AMICON, Danvers, Massachusetts) and subjected to Protein A-Sepharose 4B (Sigma, St. Louis, Missouri) chromatography. The bound proteins were eluted and subjected to Superose-12 (Pharmacia/LKB, Piscataway, New Jersey) gel filtration chromatography.

Superose-12 fractions containing LFA3TIP with the least amount of contaminating proteins, as determined on SDS-PAGE gels and by Western blot analysis, (see, e.g., Towbin et al., Proc. Natl. Acad. Sci. USA, 74, pp. 4350-54 (1979); Antibodies: A Laboratory Manual, pp. 474-510 (Cold Spring Harbor Laboratory (1988)), were pooled and concentrated in a YM30 Centricon (AMICON). LFA3TIP was detected on Western blots using a rabbit anti-LFA-3 polyclonal antiserum, followed by detectably labeled goat anti-rabbit IgG. The purified LFA3TIP of COS7 cells was a dimer of two monomeric LFA-3-Ig fusion proteins, connected by disulfide bonds.

### Pharmaceutical Compositions And Methods According To This Invention

The methods according to this invention improve tolerance of transplanted allograft tissue or xenograft tissue by administering to a mammal the graft tissue and one or more CD2 binding proteins, including derivatized forms thereof. The CD2 binding proteins may alternatively be administered as part of a pharmaceutical composition.

Useful pharmaceutical compositions will comprise one or more CD2 binding proteins, including derivatized forms thereof, typically in a pharmaceutically acceptable carrier. By "pharmaceutically acceptable carrier" is meant a carrier that does not cause an allergic reaction or other untoward effect in patients to whom it is administered.

Suitable pharmaceutically acceptable carriers include, for example, one or more of water, saline, phosphate buffered saline, dextrose, glycerol, ethanol and the like, as well as combinations thereof. Pharmaceutically acceptable carriers may further comprise minor amounts of auxiliary substances such as wetting or emulsifying agents, preservatives or buffers, which enhance the shelf life or effectiveness of the CD2 binding protein.

The CD2 binding proteins or compositions useful in this invention will preferably be administered in an "effective amount," meaning an amount capable of improving tolerance to an allograft or xenograft as defined herein.

It will be apparent to those of skill in the art that the effective amount of CD2 binding protein will depend, inter alia, upon the administration schedule, the unit dose administered, whether the CD2 binding protein is administered in combination with other therapeutic agents, the immune status and health of the patient, the therapeutic or prophylactic activity of the particular CD2 binding protein administered and its serum half-life.

The pharmaceutical compositions may further be used in conjunction with general immunosuppressive agents. These include, for example, cyclosporine, azathioprine and steroids, such as Depo-Medrol (methylprednisolone acetate), Solumederol (methylprednisolone sodium succinate), and prednisone, administered in amounts effective to suppress immune response in the mammal being treated. For example, cyclosporine may be administered at 2-25 mg/kg/day p.o. starting the day before surgery, azathioprine may be administered at 50-200 mg/day, Solumederol may be administered at 125 mg i.v. at the time of transplantation and on the first post-operative day, prednisone may be administered at 1 mg/kg/day p.o. starting on the second post-operative day or Depo-Medrol may be administered at 0.8 mg/kg/day i.m. starting on the second post-operative day. The above dosages will, of course, be varied by the practitioner depending upon factors well known to those of skill in the art. In general, when used in conjunction with an CD2 binding protein, it will be desired to use the lowest possible effective concentration of such immunosuppressive agents.

The pharmaceutical compositions may further comprise other therapeutic or prophylactic agents. The CD2 binding protein and the other active agent may be in the form of a single conjugated molecule. Conjugation of the two components may be achieved by standard cross-linking techniques well known in the art. A single molecule may also take the form of a recombinant fusion protein.

The additional immunosuppressive, therapeutic or prophylactic agents may be administered in single dosage form with the CD2 binding protein, in a multiple dosage form separately from the CD2 binding protein, but contemporaneously, or in a multiple dosage form wherein the components are administered separately but sequentially. Such combination therapies may advantageously utilize lower dosages of the immunosuppressive, therapeutic or prophylactic agents.

The pharmaceutical compositions or CD2 binding proteins may be in a variety of forms. These include, for example, solid, semi-solid and liquid dosage forms, such as tablets, pills, powders, liquid solutions, dispersions or suspensions, liposomes, suppositories, injectable and infusible solutions. The preferred form depends on the intended mode of administration and therapeutic application. The preferred form is injectable or infusible solutions.

Typically, the CD2 binding protein will be suspended in a sterile saline solution for therapeutic uses. The pharmaceutical compositions may alternatively be formulated to control release of the active ingredients or to prolong their presence in a recipient's system. Numerous suitable drug delivery systems are known and include, e.g., hydrogels, hydroxymethylcellulose, microcapsules, liposomes, microemulsions, microspheres, and the like.

A mammal that is to receive transplanted graft tissue and a CD2 binding protein is administered a dose between about 0.01 and about 10 mg CD2 binding protein per kg body weight, more preferably between about 0.01 and about 2 mg CD2 binding protein per kg body weight, and most preferably between about 0.01 and about 1 mg CD2 binding protein per kg body weight.

The CD2 binding protein or composition should be administered about once per day until, within the judgment of the practitioner, the danger of rejection of the allograft or xenograft tissue has diminished. The length of administration of the CD2 binding protein or composition is dependent upon the mammal's acceptance of the graft tissue. General clinical indications of rejection will vary with the particular organ transplanted. However, fever, malaise and organ dysfunction are typical clinical indications of rejection. Symptoms of organ dysfunction depend upon the organ transplanted, but are characterized by well known and recognized indicia to those of skill in the art.

The success of the treatment may be measured by a variety of methods including biopsies, such as incisional myocardial biopsy or percutaneous endomyocardial biopsy to determine the extent of lymphocyte infiltration, blood assays to determine the extent of lymphocytotoxic antibody production or a mixed lymphocyte reaction (see, e.g., Krensky et al., J. Immunol., 131, pp. 611-16 (1983); Bradley, "Mixed Lymphocyte Responses", in Selected Methods in Cellular Immunology (Mishell and Shiigi, eds.), pp. 162-64 (W.H. Freeman and Co., San Francisco 1980)). In the case of renal transplants, biopsies can be taken to determine the extent of mononuclear cell infiltration and proliferation, or necrosis of the arterial endothelium and media in the graft tissue. (Cosimi et al., J. Immunol., 144, pp. 4604-12 (1990)).

The method of the present invention, in a preferred embodiment for allograft tissue, comprises administering the CD2 binding protein once per day for two consecutive days before the transplant and once per day for one to ten consecutive days after the transplant. More preferably, the CD2 binding protein is administered once per day for two consecutive days before the transplant and once per day for two consecutive days after the transplant.

The method of the present invention, in a preferred embodiment for xenograft tissue, comprises administering, before the transplant, a CD2 binding protein contemporaneously with tissue from the xenograft source. As used herein, "contemporaneously" when referring to the administration of tissue from a xenograft source (other than the graft tissue) and a CD2 binding protein, will mean that their administration occurs near enough in time to allow the binding protein to bind to the tissue from the xenograft source at an effective level to inhibit a significant immune response. Preferably, the binding protein is bound to the tissue from the xenograft source at saturating levels. In a preferred embodiment of this invention, administration of one occurs within approximately zero to six hours of the other. Most preferably, the tissue from the xenograft source and the CD2 binding protein are administered within approximately zero to one hour of each other. Either may be administered first. It is preferable, however, that the binding protein be administered prior to tissue from the xenograft source.

In an alternate embodiment of the present invention, the contemporaneous administration is followed by the administration of CD2 binding protein before the transplant.

More preferably, the CD2 binding protein is administered before the xenograft transplant once per day for two consecutive days, then contemporaneously with tissue from the xenograft source once per day for one day, and then once per day for one to ten consecutive days. If the xenograft source species and recipient species are unusually discordant, it may be necessary to administer the CD2 binding protein contemporaneously with tissue from the xenograft source once per day for two consecutive days according to the above schedules. In a preferred embodiment, the binding protein is administered once per day for five to ten consecutive days after the contemporaneous administration and before the transplant according to the above schedules. Most preferably, the contemporaneous administration of the CD2 binding protein and tissue from the xenograft source is simultaneous.

Although not wishing to be bound by theory, applicants administer tissue from the xenograft source to the mammal contemporaneously with CD2 binding protein with the intent of inhibiting the development of a population of activated cells specifically reactive against that tissue. The contemporaneous administration of CD2 binding proteins induces tolerance to the specific subset of antigens carried by cells from the specific xenograft source. Accordingly, it will be understood that any tissue from the xenograft source may be appropriate, however blood cells from the xenograft source are preferred. Such tissue should be administered in an amount sufficient to elicit an immune response. The preferred method of administration of tissue from the xenograft source is intravenous. The administration of between about 1 x 10⁶ to about 1 x 10⁸ whole blood cells most preferably will serve as the tissue from the xenograft source. It will be recognized, however, that lower or higher dosages and other administration schedules may be employed.

The CD2 binding protein or pharmaceutical composition may be administered intravenously, intramuscularly, subcutaneously, intra-articularly, intrathecally, periostally, orally, topically or by inhalation. Ordinarily, intravenous (i.v.) or intramuscular (i.m.) administration will be preferred, however, more localized administrations in the area of transplantation may be more desirable in some cases due to the wide range of cells in the body that express LFA-3.

In a preferred embodiment of the method of the present invention, the graft tissue is perfused with an effective amount of LFA-3 or CD2 binding protein before implantation into the mammal. Most preferably, the graft tissue is perfused with enough LFA-3 or CD2 binding protein to saturate all CD2 or LFA-3 sites on the graft tissue before implantation into the mammal.

In order that this invention may be better understood, the following examples are set forth. These examples are for purposes of illustration only, and are not to be construed as limiting the scope of the invention in any manner.

### EXAMPLES

### Example 1

### Purification Of Anti-LFA-3 Monoclonal Antibody 1E6 and Monoclonal Antibody MOPC21

1E6 hybridoma cells (ATCC HB 10693) were grown in RPMI 1640 medium supplemented with 2% fetal calf serum, 150 µg/ml streptomycin and 50 µg/ml gentamicin (GIBCO Life Technologies, Gaithersburg, Maryland) in three 40 liter stirred glass vessels (Bellco, # 196536000) at 37°C for 7 - 10 days. The conditioned media was pooled and collected into 100 liter carboys (NALGENE). Sodium azide was added to make the pooled suspension 0.02% final concentration. The cell debris was removed through a 5 µ filter cartridge (Polygard, #CN5001E06, Millipore, Bedford, Massachusetts) followed by a 0.3 µ filter cartridge (Polygard, #CN0301E06, Millipore, Bedford, Massachusetts) at room temperature. The clarified supernatant was concentrated 50 to 100 fold using a YM30 S10 spiral filter cartridge (AMICON, Danvers, Massachusetts) at 4°C. The concentrate from 50 liters of conditioned media was diluted with two volumes of equilibration buffer (3 M glycine, 1.5 M sodium chloride, pH 8.9) and passed through 90 ml of Protein A-Sepharose (Schleicher and Schuell, Keene, New Hampshire) overnight by gravity at 4°C.

The column was washed with equilibration buffer and the bound proteins were subsequently eluted with 100 mM sodium citrate, pH 3.0. The eluted fractions were collected into 1/10 fraction volume of 1 M HEPES, pH 7.8. A280 readings of the fractions were taken and the fractions containing the eluted protein were pooled and stored at -70°C. Protein A-purified 1E6 was prepared from a total of about 200 liters of conditioned media. The various pools were thawed, combined and concentrated to about 10 mg/ml protein in a 2 liter Amicon stirred cell using a YM30 filter (AMICON, Danvers, Massachusetts). The concentrated material was divided into five 100 ml aliquots. Each aliquot was passed through a 1 liter Superose-6 gel filtration column (Pharmacia, Piscataway, New Jersey) developed in phosphate buffered saline at room temperature. The peak fractions containing 1E6 were pooled and stored at -70°C. When all the material was processed, the pools were thawed, combined and adjusted to 2-3 mg/ml protein with phosphate buffered saline. The final material was divided into 15 ml aliquots and stored at -70°C until use.

MOPC21 was purified from ascites purchased from the Sigma Chemical Corporation (St. Louis, Missouri) by diluting the ascites into the "Protein A loading buffer" of 3 M glycine, 1.5 M sodium chloride, pH 8.9, and passing it over 25 ml of Protein A-Sepharose (Schleicher and Schuell, Keene, New Hampshire) at room temperature. The column was washed with the loading buffer until the optical density at 280 nm returned to a baseline level. The bound IgG was eluted with 50 mM sodium acetate, pH 3.0, at room temperature and dialyzed overnight against 50 volumes of phosphate buffered saline at 4°C. After dialysis, the MOPC21 was passed through a 1 liter Superose-6 gel filtration column (Pharmacia, Piscataway, New Jersey) developed in phosphate buffered saline at room temperature. The peak fractions containing MOPC21, were pooled, adjusted with phosphate buffered saline to a final concentration of 2 mg/ml protein and stored in 30 mg aliquots at -70°C until use. All preparations contained less than 10 units/ml endotoxin as determined using the commercially available kit Chromogenic LAL (Whittaker M.A. Bioproducts, Walkersville, Maryland). Except as otherwise noted, all purification steps were performed at room temperature.

### Example 2

### Effect Of Administration Of Anti-LFA-3 Monoclonal Antibody 1E6 On Lymphocyte Function

### A. Administration And Sampling Protocols

Two outbred, adult baboons A and B (Papio anubis) were given bolus injections of 1.45 mg/kg of the purified anti-LFA-3 monoclonal antibody 1E6, i.v., by portacatheter once daily for five consecutive days. Baboon A weighed 12 kg. Baboon B weighed 9.5 kg. As a control, another adult baboon C, 9.4 kg, was injected with equal amounts of the non-specific, isotype-matched mouse monoclonal antibody MOPC21 (Sigma Chemical Corp., St. Louis, Missouri). Blood was drawn from the baboons once or twice before the first injection of antibody and then, daily for five days, four hours after each injection. Blood was also drawn on day 8, day 11 and day 14, where day 1 is the day of the first injection. This administration and sampling protocol was used for all of the assays described in this example, unless otherwise stated.

### B. Toxicology Study with Anti-LFA-3 Monoclonal Antibody 1E6

The general toxicity of anti-LFA-3 monoclonal antibody 1E6 and the potential effect on the physical condition, hematology and blood chemistry of baboons was evaluated. The general physical condition of the baboons remained unchanged throughout the study. No obvious or immediate side effects could be observed. Hematology and blood chemistries generally remained normal. In particular, Na⁺, Cl⁻, K⁺, creatine, blood urea nitrogen and liver enzymes AST and ALT levels all remained with normal limits. In addition, blood cell counts, including hematocrit, white blood cells, lymphocytes, monocytes, segmented neutrophils and eosinophils, generally stayed within normal ranges. However, baboon B showed a substantial decrease in segmented neutrophils after day five.

### C. Serum Levels of Anti-LFA-3 Monoclonal Antibody 1E6 and Control MOPC21

Serum was prepared from blood drawn four hours after antibody injection. For the baboons injected with 1E6 (baboons A and B), additional serum was collected at the 24 hour time point, just before the antibody injections on days one to five. Serum was also collected on days 8, 11 and 14. Serum levels of MOPC21 and 1E6 were determined by measurement of mouse IgG levels with an ELISA using microtiter plates coated with goat anti-mouse IgG (Jackson Immunoresearch, Malvern, Pennsylvania). These ELISAs were standardized using MOPC21 and 1E6 purified as described in Example 1. Serum levels of 1E6 capable of binding to LFA-3 (i.e., "active" 1E6) were measured with an ELISA using microtiter plates coated with a soluble LFA-3 polypeptide consisting of AA₁-AA₁₈₄ of LFA-3 (see U.S. patent 4,956,281, which is herein incorporated by reference). This ELISA was also standardized with 1E6 purified as described in Example 1. In all of the above ELISA assays, binding of 1E6 or MOPC21 to microtiter plates was detected using a second goat anti-mouse antibody that was labelled with alkaline phosphatase (Jackson Immunoresearch, Malvern, Pennsylvania). The bound immunoglobulin was quantified by the colorimetric conversion of the alkaline phosphatase substrate pNPP to its colored product using a Thermomax (Molecular Devices, Palo Alto, California). The ELISA reader was at a wavelength of 405 nm. (Data not shown.)

Serum levels of 1E6 and MOPC21 peaked between day four and day five (about 40-80 µg/ml antibody) and returned to pre-injection levels between day eight and day eleven. Serum levels of 1E6, 24 hours after injection, consistently decreased between 50% and 80% of the level at four hours after injection for serum collected on days 1-5. In comparison, MOPC21 levels decreased only between 10% and 20% after 24 hours. The percentage of active 1E6 in serum varied between 40% and 70%. 1E6 serum levels were higher in baboon B as compared to baboon A (9.5 kg compared to 12 kg body weight), possibly as a result of different tissue space distribution.

The titer of anti-1E6 antibodies in the treated baboon serum was determined by ELISA. Purified 1E6 was coupled to microtiter plates and serum from each bleed was assayed at increasing dilutions. (Data not shown.)

In both 1E6 injected baboons A and B, anti-1E6 antibodies were detected after the injection as early as day eleven. Anti-MOPC21 titers were detected using anti-mouse IgG coated assay plates and showed the same kinetics as anti-1E6. (Data not shown.)

### D. T cell Activation Assays In Vitro

To determine the effect of 1E6 injections on T cell activation in vitro, peripheral blood lymphocytes were isolated from antibody-injected baboons and assayed for T cell dependent B cell activation and for T cell proliferation in response to phytohemagglutinin or activating anti-CD2 monoclonal antibodies. For each of these assays, peripheral blood lymphocytes were isolated on Ficoll-Hypaque (Pharmacia, Piscataway, New Jersey), according to the manufacturer's suggested protocol. Peripheral blood lymphocytes were stored overnight in tissue culture medium containing 10% fetal calf serum at room temperature prior to each assay.

### 1. T cell Dependent B-Cell Activation Assay

The T cell dependent B cell activation to immunoglobulin secretion can be blocked by anti-LFA-3 antibodies (MOPC21 is used as a control).

Peripheral blood mononuclear cells were purified from whole blood on Ficoll Hypaque density medium (Pharmacia, Piscataway, New Jersey), according to the manufacturer's instructions. Adherent macrophages were removed by incubating the mononuclear cells on plastic dishes for 45 minutes at 37°C. The nonadherent lymphocytes were washed in a physiologically compatible culture medium (RPMI 1640, GIBCO Life Technologies, Gaithersburg, Maryland), determined to contain minimal macrophages by FACS analysis on a FACStar (Becton Dickinson Corporation, Mountainview, California) using fluorescently labelled antibodies specific for macrophage/monocyte cell surface antigens and cultured in 96-well round bottom plates (RPMI 1640 supplemented with 10% fetal calf serum, 2 mM glutamine, 5 x 10-5 M β-mercaptoethanol and nonessential amino acids (GIBCO Life Technologies, Gaithersburg, Maryland)).

In this culture, T cells activate B cells to secrete immunoglobulin. The B cells are not activated in the absence of T cells. The immunoglobulin secreted into the culture medium was measured by sampling culture medium on day seven and day twelve after the initiation of the culture. The supernatant (cell free) samples were analyzed for baboon immunoglobulin using an ELISA in which the assay plates were coated with goat anti-human immunoglobulin (Jackson Immunoresearch, Malvern, Pennsylvania), which also recognizes baboon immunoglobulin, but does not bind to immunoglobulin present in the fetal calf serum or to mouse immunoglobulins. The immunoglobulins from the culture supernatants that were bound to the goat anti-human immunoglobulin-coated plates were detected using a second goat anti-human immunoglobulin reagent to which an enzyme, alkaline phosphatase, had been coupled (Jackson Immunoresearch, Malvern, Pennsylvania). The bound immunoglobulin was quantified by the colorimetric conversion of the alkaline phosphatase substrate pNPP (para-nitrophenylphosphate) to its colored product. Substrate conversion was measured in a Thermomax (Molecular Devices, Palo Alto, California) ELISA reader at a wavelength of 405 nm.

The results of these experiments are shown in Figures 1 and 2. Figure 1 displays relative absorbance units at 405 nm from the ELISA assay for assays performed on baboon B (1E6) lymphocytes from days 0, 1-5, 8, 11 and 14. Figure 2 displays relative absorbance units at 405 nm from the ELISA assay for assays performed on lymphocytes from baboons A (1E6) and C (MOPC21) on days 0, 1-5, 8 and 11.

For baboon B, T cell dependent B cell Ig production decreased on the second day of 1E6 injections and remained at about 35% of the day zero value through day eleven (Figure 1).

For baboon A, Ig production was higher on days 1-11 as compared to the level before the injection. This is likely due to the lower 1E6 serum level achieved in baboon A versus baboon B. If Ig production levels observed on days one through four are taken as a base value, then a 40% inhibition of Ig secretion was observed on day five, and a 20% inhibition on day eleven (Figure 2).

In baboon C, after injection with MOPC21, peripheral blood lymphocytes showed increased levels of Ig production between days two and eleven as compared to the level on day zero.

### 2. T cell Proliferation Assay

In a T cell proliferation assay, we measured the ability of activating anti-CD2 monoclonal antibodies or phytohemagglutinin ("PHA") to cause proliferation of T cells isolated from baboons A, B and C on days 0, 1-5, 8, 11 and 14. 1 x 10⁵ peripheral blood lymphocytes per well were incubated (1) with anti-CD2 monoclonal antibodies T11₁ and T11₃ at a 1:900 dilution of ascites fluid, (2) in medium alone, or (3) with PHA (Sigma Chemical Corporation, St. Louis, Missouri) (10 µg/ml) for three days. After three days, cells were labelled with 1 µCi/well ³HdT for 18 hours and then harvested. (Data not shown.)

Peripheral blood lymphocytes from baboon B showed no increase of ³HdT incorporation in response to activating anti-CD2 monoclonal antibodies and very low proliferative activity in medium on days zero to fourteen.

Peripheral blood lymphocytes from baboon A responded to anti-CD2 monoclonal antibodies and PHA. After day four, proliferation in response to those agents was inhibited about nine fold and remained low until at least day fourteen.

Peripheral blood lymphocytes from baboon C, the MOPC21 control, showed very low proliferative activity at all time points tested, under all conditions.

The significance of the data obtained is not clear because of irreproducibility of T cell proliferation in baboon C and day zero results for baboons A, B and C.

### Example 3

### Effect Of Administration Of LFA3TIP On Lymphocyte Function

### A. Administration And Sampling Protocols

Two outbred, adult baboons (4.6 and 7.4 kg) (Papio anubis) were given bolus injections of 3 mg/kg of purified LFA3TIP (obtained from Biogen, Inc., Cambridge, Massachusetts), i.v., by portacatheter once daily for five consecutive days. Blood was drawn from the baboons once before the first injection of antibody and then, daily for five days, 24 hours after each injection. Blood was also drawn on day 8, day 10, day 15, and day 22, where day 1 is the day of the first injection. This administration and sampling protocol was used for all of the assays described in this example, unless otherwise stated.

### B. Toxicology Study With LFA3TIP

The general toxicity of LFA3TIP and its potential effect on the physical condition, hematology and blood chemistry of baboons were evaluated. The general physical condition of the baboons remained unchanged throughout the study. No obvious or immediate side effects could be observed. Hematology and blood chemistries generally remained normal. In particular, Na⁺, Cl⁻, K⁺, creatine, blood urea nitrogen and liver enzymes AST and ALT levels all remained within normal limits. In addition, blood cell counts, including hematocrit, white blood cells, lymphocytes, monocytes, segmented neutrophils, and eosinophils, generally stayed within normal ranges. The ratio of CD4/CD8 expressing cells also stayed within normal ranges.

Plasma levels of LFA3TIP 10 days after the last injection were still about 32% of the LFA3TIP levels immediately following the last injection, which indicates a much longer half-life than generally observed with murine monoclonal antibodies. Fluorescent labeling of CD4 and CD8 expressing cells indicated that about 10% of CD4⁺ cells and about 90% of CD8⁺ cells were still coated with LFA3TIP 10 days after the last injection.

### Example 4

### Baboon Cardiac Allograft Model

### A. 1E6 Treatment

An experimental primate cardiac allograft model where baboon hearts were transplanted heterotopically in a nonfunctioning position into the necks of ABO-matched outbred baboons (Papio anubis) was used to assess the effect of anti-LFA-3 monoclonal antibody 1E6 on allograft rejection. The protocol used was substantially as described in Michler et al., "Techniques For Primate Heterotopic Cardiac Xenotransplantation," J. Med. Primatol., 14, pp. 357-62 (1985), except that an allograft not a xenograft was performed.

Purified 1E6 prepared as described above was injected into one adult baboon (weight 32 kg) at a dose of 5 mg/kg, starting on day one, for 2 consecutive days before the transplant. On the third day, a cardiac heterotopic allograft transplant was performed with a heart from a young, 3 kg baboon. One dose of 5 mg/kg of 1E6 was injected on the day of the transplant and then once a day for ten consecutive days. Blood samples were collected two days before transplantation, prior to injection. Blood samples were also collected coincident with transplantation and on the fifth, tenth, sixteenth, nineteenth and twenty-first day after transplantation. An assay for total 1E6 serum levels and the proportion of active 1E6 in the serum, i.e., the percentage of 1E6 capable of binding to LFA-3, was performed as described in Example 2C. No general immunosuppressive agents were administered to the baboon.

The graft was palpated on a daily basis and monitored by palpation and visual assessment of heart beat. Electrocardiograms were performed on a weekly basis. A percutaneous endomyocardial biopsy was performed on the sixteenth day after transplantation. All blood chemistry and cell counts performed on the above described blood samples were within the normal limits.

Untreated control cardiac allografts in this model system were rejected a mean of 9±3 days (n=5) after implantation in non-immunosuppressed baboons (Rose et al., "Cardiac Xenotransplantation", Progress In Cardiovascular Diseases, 33, pp. 105-17 (1990)). Rejection is defined, for the purposes of this model system, as swelling and hardening of the heart, and cessation of heart beat as measured by an electrocardiogram. In addition, progressive infiltration of lymphocytes in the myocardium, production of lymphocytotoxic antibodies and reaction to donor peripheral blood lymphocytes are monitored. Survival of a graft in this system for longer than nine days, without immunosuppressive therapy, indicates an increased level of tolerance.

In the 1E6 treated baboon, the transplanted allogeneic heart was still beating twenty-three days after the transplant. Thus, 1E6 dramatically improved tolerance for a cardiac allograft.

### B. LFA3TIP Treatment

Using procedures substantially as described in Example 4A, the effect of LFA3TIP on cardiac allograft rejection is assessed. Purified LFA3TIP (described supra) is injected into one adult baboon at a dose of 3 mg/kg on day one for 2 consecutive days before the transplant. On the third day a cardiac heterotopic allograft transplant is performed with a heart from a young baboon. One dose of 3 mg/kg LFA3TIP is injected on the day of the transplant and then once a day for nine consecutive days.

The schedule of blood sample collection and analysis, and assessment of allograft rejection, is substantially as described in Example 4A.

Survival of the graft in the baboon that is treated with LFA3TIP is extended, compared to graft survival in untreated baboons, indicating increased graft tolerance due to LFA3TIP.

### Deposits

Murine hybridoma cells and antibodies useful in the present invention are exemplified by cultures deposited under the Budapest Treaty with American Type Culture Collection, Rockville, Maryland, U.S.A., on March 5. 1991. and identified as:

| Designation | ATCC Accession No. |
|---|---|
| 1E6 | HB 10693 |
| HC-1B11 | HB 10694 |
| 7A6 | HB 10695 |
| 8B8 | HB 10696 |

E. coli JA221 transformed with plasmid pSAB152 (encoding LFA3TIP) was deposited under the Budapest Treaty with American Type Culture Collection on October 1, 1991 and identified as:

| Designation | ATCC Accession No. |
|---|---|
| pSAB152 | 68720 |

A bacteriophage carrying a plasmid encoding transmembrane LFA-3 was deposited under the Budapest Treaty with In Vitro International, Inc., Linthicum, Maryland, U.S.A., on May 28, 1987. This deposit was transferred to American Type Culture Collection on June 20, 1991 and identified as:

| Designation | ATCC Accession No. |
|---|---|
| λHT16[λgt10/LFA-3] | 75107 |

E. coli transformed with a plasmid encoding PI-linked LFA-3 was deposited under the Budapest Treaty with In Vitro International, Inc. on July 22, 1918. This deposit was transferred to American Type Culture Collection on June 20, 1991 and identified as:

| Designation | ATCC Accession No. |
|---|---|
| p24 | 68788 |

### Sequences

The following is a summary of the sequences set forth in the Sequence Listing:

While we have hereinbefore described a number of embodiments of this invention, it is apparent that our basic embodiments can be altered to provide other embodiments that utilize the processes of this invention. Therefore, it will be appreciated that the scope of this invention includes all alternative embodiments and variations which are defined in the foregoing specification and by the claims appended hereto; and the invention is not to be limited by the specific embodiments that have been presented herein by way of example.

## Claims

1. The use of a protein that binds CD2, which is a derivative of a soluble LFA-3 polypeptide, the derivative being an immunoglobulin fusion comprising the soluble LFA-3 polypeptide fused to an immunoglobulin region in the preparation of a medicament for use in a method of improving tolerance of transplanted allograft tissue or xenograft tissue in a mammal, including a human, wherein the method comprises implanting in the mammal an allograft or a xenograft and administering the protein.

2. The use as claimed in claim 1, wherein the CD2 binding protein is an immunoglobulin fusion which comprises the soluble LFA-3 polypeptide linked to a human immunoglobulin heavy chain hinge region and constant region, or portions thereof.

3. The use as claimed in claim 2, wherein the soluble LFA-3 polypeptide is selected from the group consisting of AA₁-AA₉₂ of SEQ. ID NO:2. AA₁-AA₈₀ of SEQ ID NO:2, AA₅₀-AA₆₅ or SEQ ID NO:2 and AA₂₀-AA₈₀ of SEQ ID NO 2.

4. The use as claimed in claim 2, wherein the soluble LFA-3 polypeptide is selected from the group consisting of AA₁-AA₉₂ of SEQ. ID NO:2.

5. The use as claimed in claim 2, wherein the soluble LFA-3 polypeptide is selected from the group consisting of AA₁-AA₃₁₉ of SEQ ID NO:8.

6. The use as claimed in claim 5, wherein the CD2 binding protein is LFA-3TIP.

## Patentansprüche

1. Verwendung eines CD2-bindenden Proteins, das ein Derivat eines löslichen LFA-3 Polypeptides ist, wobei das Derivat ein Immunglobulin-Fusionsprotein ist, umfassend das lösliche LFA-3 Polypeptid verbunden mit einer Immunglobulin-Region, für die Herstellung eines Medikamentes zur Verwendung in einem Verfahren zur Verbesserung der Toleranz von transplantiertem allogenem Gewebe oder xenogenem Gewebe in einem Säuger, einschließlich dem Menschen, wobei das Verfahren das Implantieren eines allogenen oder eines xenogenen Transplantates in den Säuger und die Verabreichung des Proteins umfasst.

2. Verwendung nach Anspruch 1, wobei das CD2-bindende Protein ein Immunglobulin-Fusionsprotein ist, umfassend das lösliche LFA-3 Polypeptid verknüpft mit einer Gelenkregion ("hinge region") und konstanten Region der schweren Kette eines menschlichen Immunglobulins, oder Teilen davon.

3. Verwendung nach Anspruch 2, wobei das lösliche LFA-3 Polypeptid ausgewählt ist aus der Gruppe bestehend aus AA₁-AA₉₂ der SEQ ID NO:2, AA₁-AA₈₀ der SEQ ID NO:2, AA₅₀-AA₆₅ der SEQ ID NO:2 und AA₂₀-AA₈₀ der SEQ ID NO:2.

4. Verwendung nach Anspruch 2, wobei das lösliche LFA-3 Polypeptid ausgewählt ist aus der Gruppe bestehend aus AA₁-AA₉₂ der SEQ ID NO:2.

5. Verwendung nach Anspruch 2, wobei das lösliche LFA-3 Polypeptid ausgewählt ist aus der Gruppe bestehend aus AA₁-AA₃₁₉ der SEQ ID NO:8.

6. Verwendung nach Anspruch 5, wobei das CD2-bindende Protein LFA-3TIP ist.

## Revendications

1. Utilisation d'une protéine qui lie la CD2, qui est un dérivé du polypeptide soluble LFA-3, le dérivé étant une fusion de l'immunoglobuline comprenant le polypeptide soluble LFA-3 fusionné à une zone de l'immunoglobuline dans la préparation d'un médicament destiné à être utilisé dans un procédé d'amélioration de la tolérance à un tissu de greffe allogène ou xénogène chez un mammifère, l'homme y compris, dans laquelle le procédé comprend l'étape consistant à implanter chez le mammifère une allogreffe ou une xénogreffe et à administrer la protéine.

2. Utilisation selon la revendication 1, dans laquelle la protéine liant la CD2 est une fusion de l'immunoglobuline qui comprend le polypeptide soluble LFA-3 lié à la zone charnière et à la zone constante d'une chaîne lourde d'immunoglobuline humaine, ou à des portions de celles-ci.

3. Utilisation selon la revendication 2, dans laquelle le polypeptide soluble LFA-3 est sélectionné parmi le groupe se composant de AA₁-AA₉₂ de SEQ.ID NO:2. AA₁-AA₈₀ de SEQ ID NO:2, AA₅₀-AA₆₅ de SEQ ID NO:2 et AA₂₀-AA₈₀ de SEQ ID NO 2.

4. Utilisation selon la revendication 2, dans laquelle le polypeptide soluble LFA-3 est sélectionné parmi le groupe se composant de AA₁-AA₉₂ de SEQ.ID NO:2.

5. Utilisation selon la revendication 2, dans laquelle le polypeptide soluble LFA-3 est sélectionné parmi le groupe se composant de AA₁-AA₃₁₉ de SEQ ID NO:8.

6. Utilisation selon la revendication S, dans laquelle la protéine liant la CD2 est une LFA-3TIP.


### SEQUENCE LISTING

(1) GENERAL INFORMATION:
   (i) APPLICANT: WALLNER, Barbara P.
      BENJAMIN, Christopher D.
   (ii) TITLE OF INVENTION: METHODS OF IMPROVING ALLOGRAFT OR XENOGRAFT TOLERANCE BY ADMINISTRATION OF LFA-3 OR CD2 BINDING PROTEINS
   (iii) NUMBER OF SEQUENCES: 8
   (iv) CORRESPONDENCE ADDRESS:
      (A) ADDRESSEE: c\o FISH & NEAVE
      (B) STREET: 875 Third Avenue
      (C) CITY: New York
      (D) STATE: New York
      (E) COUNTRY: U.S.A.
      (F) ZIP: 10022
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 07/772,705
      (B) FILING DATE: 07-OCT-1991
   (viii) ATTORNEY/AGENT INFORMATION:
      (A) NAME: Haley Jr., James F.
      (B) REGISTRATION NUMBER: 27,794
      (C) REFERENCE/DOCKET NUMBER: B162CIP
   (ix) TELECOMMUNICATION INFORMATION:
      (A) TELEPHONE: (212) 715-0600
(2) INFORMATION FOR SEQ ID NO:1:
   (i) SEQUENCE CHARACTERISTICS:
      (A) LENGTH: 753 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..750
   (ix) FEATURE:
      (A) NAME/KEY: sig_peptide
      (B) LOCATION: 1..84
   (ix) FEATURE:
      (A) NAME/KEY: mat_peptide
      (B) LOCATION: 85..750
   (ix) FEATURE:
      (A) NAME/KEY: misc_feature
      (B) LOCATION: 1..750
      (D) OTHER INFORMATION: /note- "Human transmembrane LFA-3"
   (ix) FEATURE:
      (A) NAME/KEY: misc_feature
      (B) LOCATION: 646..714
      (D) OTHER INFORMATION: /note- "Transmembrane domain"
   (xi) SEQUENCE DESCRIPTION: SEQ ID NO:1:
   ATG GTT GCT GGG AGC GAC GCG GGG CGG GCC CTG GGG GTC CTC AGC GTG        48
   Met Val Ala Gly Ser Asp Ala Gly Arg Ala Leu Gly Val Leu Ser Val
   -28         -25                 -20                 -15
   GTC TGC CTG CTG CAC TGC TTT GGT TTC ATC AGC TGT TTT TCC CAA CAA        96
   Val Cys Leu Leu His Cys Phe Gly Phe Ile Ser Cys Phe Ser Gln Gln
           -10                  -5                   1
   ATA TAT GGT GTT GTG TAT GGG AAT GTA ACT TTC CAT GTA CCA AGC AAT       144
   Ile Tyr Gly Val Val Tyr Gly Asn Val Thr Phe His Val Pro Ser Asn
     5                  10                  15                  20
   GTG CCT TTA AAA GAG GTC CTA TGG AAA AAA CAA AAG GAT AAA GTT GCA       192
   Val Pro Leu Lys Glu Val Leu Trp Lys Lys Gln Lys Asp Lys Val Ala
                    25                  30                  35
   GAA CTG GAA AAT TCT GAA TTC AGA GCT TTC TCA TCT TTT AAA AAT AGG       240
   Glu Leu Glu Asn Ser Glu Phe Arg Ala Phe Ser Ser Phe Lys Asn Arg
                40                  45                  50
   GTT TAT TTA GAC ACT GTG TCA GGT AGC CTC ACT ATC TAC AAC TTA ACA       288
   Val Tyr Leu Asp Thr Val Ser Gly Ser Leu Thr Ile Tyr Asn Leu Thr
            55                  60                  65
   TCA TCA GAT GAA GAT GAG TAT GAA ATG GAA TCG CCA AAT ATT ACT GAT       336
   Ser Ser Asp Glu Asp Glu Tyr Glu Met Glu Ser Pro Asn Ile Thr Asp
        70                  75                  80
   ACC ATG AAG TTC TTT CTT TAT GTG CTT GAG TCT CTT CCA TCT CCC ACA       384
   Thr Met Lys Phe Phe Leu Tyr Val Leu Glu Ser Leu Pro Ser Pro Thr
    85                  90                  95                 100
   CTA ACT TGT GCA TTG ACT AAT GGA AGC ATT GAA GTC CAA TGC ATG ATA      432
   Leu Thr Cys Ala Leu Thr Asn Gly Ser Ile Glu Val Gln Cys Met Ile
                   105                 110                 115
   CCA GAG CAT TAC AAC AGC CAT CGA GGA CTT ATA ATG TAC TCA TGG GAT      480
   Pro Glu His Tyr Asn Ser His Arg Gly Leu Ile Met Tyr Ser Trp Asp
               120                 125                 130
   TGT CCT ATG GAG CAA TGT AAA CGT AAC TCA ACC AGT ATA TAT TTT AAG      528
   Cys Pro Met Glu Gln Cys Lys Arg Asn Ser Thr Ser Ile Tyr Phe Lys
           135                 140                 145
   ATG GAA AAT GAT CTT CCA CAA AAA ATA CAG TGT ACT CTT AGC AAT CCA      576
   Met Glu Asn Asp Leu Pro Gln Lys Ile Gln Cys Thr Leu Ser Asn Pro
       150                 155                 160
   TTA TTT AAT ACA ACA TCA TCA ATC ATT TTG ACA ACC TGT ATC CCA AGC      624
   Leu Phe Asn Thr Thr Ser Ser Ile Ile Leu Thr Thr Cys Ile Pro Ser
   165                 170                 175                 180
   AGC GGT CAT TCA AGA CAC AGA TAT GCA CTT ATA CCC ATA CCA TTA GCA      672
   Ser Gly His Ser Arg His Arg Tyr Ala Leu Ile Pro Ile Pro Leu Ala
                   185                 190                 195
   GTA ATT ACA ACA TGT ATT GTG CTG TAT ATG AAT GGT ATT CTG AAA TGT      720
   Val Ile Thr Thr Cys Ile Val Leu Tyr Met Asn Gly Ile Leu Lys Cys
               200                 205                 210
   GAC AGA AAA CCA GAC AGA ACC AAC TCC AAT TGA                          753
   Asp Arg Lys Pro Asp Arg Thr Asn Ser Asn
           215                 220
(2) INFORMATION FOR SEQ ID NO:2:
   (i) SEQUENCE CHARACTERISTICS:
      (A) LENGTH: 250 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                 -5                  1
   Ile Tyr Gly Val Val Tyr Gly Asn Val Thr Phe His Val Pro Ser Asn
    5                  10                  15                  20
   Val Pro Leu Lys Glu Val Leu Trp Lys Lys Gln Lys Asp Lys Val Ala
                   25                  30                  35
   Glu Leu Glu Asn Ser Glu Phe Arg Ala Phe Ser Ser Phe Lys Asn Arg
               40                  45                  50
   Val Tyr Leu Asp Thr Val Ser Gly Ser Leu Thr Ile Tyr Asn Leu Thr
           55                  60                  65
   Ser Ser Asp Glu Asp Glu Tyr Glu Met Glu Ser Pro Asn Ile Thr Asp
       70                  75                  80
   Thr Met Lys Phe Phe Leu Tyr Val Leu Glu Ser Leu Pro Ser Pro Thr
   85                  90                  95                  100
   Leu Thr Cys Ala Leu Thr Asn Gly Ser Ile Glu Val Gln Cys Met Ile
                   105                 110                 115
   Pro Glu His Tyr Asn Ser His Arg Gly Leu Ile Met Tyr Ser Trp Asp
               120                 125                 130
   Cys Pro Met Glu Gln Cys Lys Arg Asn Ser Thr Ser Ile Tyr Phe Lys
           135                 140                 145
   Met Glu Asn Asp Leu Pro Gln Lys Ile Gln Cys Thr Leu Ser Asn Pro
       150                 155                 160
   Leu Phe Asn Thr Thr Ser Ser Ile Ile Leu Thr Thr Cys Ile Pro Ser
   165                 170                 175                 180
   Ser Gly His Ser Arg His Arg Tyr Ala Leu Ile Pro Ile Pro Leu Ala
                   185                 190                 195
   Val Ile Thr Thr Cys Ile Val Leu Tyr Met Asn Gly Ile Leu Lys Cys
               200                 205                 210
   Asp Arg Lys Pro Asp Arg Thr Asn Ser Asn
           215                 220
(2) INFORMATION FOR SEQ ID NO:3:
   (i) SEQUENCE CHARACTERISTICS:
      (A) LENGTH: 723 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..720
   (ix) FEATURE:
      (A) NAME/KEY: sig_peptide
      (B) LOCATION: 1..84
   (ix) FEATURE:
      (A) NAME/KEY: mat_peptide
      (B) LOCATION: 85..720
   (ix) FEATURE:
      (A) NAME/KEY: misc_feature
      (B) LOCATION: 1..720
      (D) OTHER INFORMATION: /note- "Human PI-linked LFA-3"
   (ix) FEATURE:
      (A) NAME/KEY: misc_feature
      (B) LOCATION: 568..720
      (D) OTHER INFORMATION: /note- "Signal sequence for PI-linkage"
   (xi) SEQUENCE DESCRIPTION: SEQ ID NO:3:
   ATG GTT GCT GGG AGC GAC GCG GGG CGG GCC CTG GGG GTC CTC AGC GTG      48
   Met Val Ala Gly Ser Asp Ala Gly Arg Ala Leu Gly Val Leu Ser Val
   -28         -25                 -20                 -15
   GTC TGC CTG CTG CAC TGC TTT GGT TTC ATC AGC TGT TTT TCC CAA CAA      96
   Val Cys Leu Leu His Cys Phe Gly Phe Ile Ser Cys Phe Ser Gln Gln
           -10                 -5                  1
   ATA TAT GGT GTT GTG TAT GGG AAT GTA ACT TTC CAT GTA CCA AGC AAT     144
   Ile Tyr Gly Val Val Tyr Gly Asn Val Thr Phe His Val Pro Ser Asn
    5                  10                  15                  20
   GTG CCT TTA AAA GAG GTC CTA TGG AAA AAA CAA AAG GAT AAA GTT GCA     192
   Val Pro Leu Lys Glu Val Leu Trp Lys Lys Gln Lys Asp Lys Val Ala
                   25                  30                  35
   GAA CTG GAA AAT TCT GAA TTC AGA GCT TTC TCA TCT TTT AAA AAT AGG     240
   Glu Leu Glu Asn Ser Glu Phe Arg Ala Phe Ser Ser Phe Lys Asn Arg
               40                  45                  50
   GTT TAT TTA GAC ACT GTG TCA GGT AGC CTC ACT ATC TAC AAC TTA ACA     288
   Val Tyr Leu Asp Thr Val Ser Gly Ser Leu Thr Ile Tyr Asn Leu Thr
           55                  60                  65
   TCA TCA GAT GAA GAT GAG TAT GAA ATG GAA TCG CCA AAT ATT ACT GAT     336
   Ser Ser Asp Glu Asp Glu Tyr Glu Met Glu Ser Pro Asn Ile Thr Asp
       70                  75                  80
   ACC ATG AAG TTC TTT CTT TAT GTG CTT GAG TCT CTT CCA TCT CCC ACA     384
   Thr Met Lys Phe Phe Leu Tyr Val Leu Glu Ser Leu Pro Ser Pro Thr
   85                  90                  95                  100
   CTA ACT TGT GCA TTG ACT AAT GGA AGC ATT GAA GTC CAA TGC ATG ATA     432
   Leu Thr Cys Ala Leu Thr Asn Gly Ser Ile Glu Val Gln Cys Met Ile
                   105                 110                 115
   CCA GAG CAT TAC AAC AGC CAT CGA GGA CTT ATA ATG TAC TCA TGG GAT     480
   Pro Glu His Tyr Asn Ser His Arg Gly Leu Ile Met Tyr Ser Trp Asp
               120                 125                 130
   TGT CCT ATG GAG CAA TGT AAA CGT AAC TCA ACC AGT ATA TAT TTT AAG         528
   Cys Pro Met Glu Gln Cys Lys Arg Asn Ser Thr Ser Ile Tyr Phe Lys
           135                 140                 145
   ATG GAA AAT GAT CTT CCA CAA AAA ATA CAG TGT ACT CTT AGC AAT CCA         576
   Met Glu Asn Asp Leu Pro Gln Lys Ile Gln Cys Thr Leu Ser Asn Pro
       150                 155                 160
   TTA TTT AAT ACA ACA TCA TCA ATC ATT TTG ACA ACC TGT ATC CCA AGC         624
   Leu Phe Asn Thr Thr Ser Ser Ile Ile Leu Thr Thr Cys Ile Pro Ser
   165                 170                 175                 180
   AGC GGT CAT TCA AGA CAC AGA TAT GCA CTT ATA CCC ATA CCA TTA GCA         672
   Ser Gly His Ser Arg His Arg Tyr Ala Leu Ile Pro Ile Pro Leu Ala
                   185                 190                 195
   GTA ATT ACA ACA TGT ATT GTG CTG TAT ATG AAT GGT ATG TAT GCT TTT         720
   Val Ile Thr Thr Cys Ile Val Leu Tyr Met Asn Gly Met Tyr Ala Phe
               200                 205                 210
   TAA                                                                     723
(2) INFORMATION FOR SEQ ID NO:4:
   (i) SEQUENCE CHARACTERISTICS:
      (A) LENGTH: 240 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                 -5                  1
   Ile Tyr Gly Val Val Tyr Gly Asn Val Thr Phe His Val Pro Ser Asn
    5                  10                  15                  20
   Val Pro Leu Lys Glu Val Leu Trp Lys Lys Gln Lys Asp Lys Val Ala
                   25                  30                  35
   Glu Leu Glu Asn Ser Glu Phe Arg Ala Phe Ser Ser Phe Lys Asn Arg
               40                  45                  50
   Val Tyr Leu Asp Thr Val Ser Gly Ser Leu Thr Ile Tyr Asn Leu Thr
           55                  60                  65
   Ser Ser Asp Glu Asp Glu Tyr Glu Met Glu Ser Pro Asn Ile Thr Asp
       70                  75                  80
   Thr Met Lys Phe Phe Leu Tyr Val Leu Glu Ser Leu Pro Ser Pro Thr
   85                  90                  95                  100
   Leu Thr Cys Ala Leu Thr Asn Gly Ser Ile Glu Val Gln Cys Met Ile
                   105                 110                 115
   Pro Glu His Tyr Asn Ser His Arg Gly Leu Ile Met Tyr Ser Trp Asp
               120                 125                 130
   Cys Pro Met Glu Gln Cys Lys Arg Asn Ser Thr Ser Ile Tyr Phe Lys
           135                 140                 145
   Met Glu Asn Asp Leu Pro Gln Lys Ile Gln Cys Thr Leu Ser Asn Pro
       150                 155                 160
   Leu Phe Asn Thr Thr Ser Ser Ile Ile Leu Thr Thr Cys Ile Pro Ser
   165                 170                 175                 180
   Ser Gly His Ser Arg His Arg Tyr Ala Leu Ile Pro Ile Pro Leu Ala
                   185                 190                 195
   Val Ile Thr Thr Cys Ile Val Leu Tyr Met Asn Gly Met Tyr Ala Phe
               200                 205                 210
(2) INFORMATION FOR SEQ ID NO:5:
   (i) SEQUENCE CHARACTERISTICS:
      (A) LENGTH: 1056 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..1053
   (ix) FEATURE:
      (A) NAME/KEY: sig_peptide
      (B) LOCATION: 1..72
   (ix) FEATURE:
      (A) NAME/KEY: mat_peptide
      (B) LOCATION: 73..1053
   (ix) FEATURE:
      (A) NAME/KEY: misc_feature
      (B) LOCATION: 1..1053
      (D) OTHER INFORMATION: /note- "Human CD2"
   (ix) FEATURE:
      (A) NAME/KEY: misc_feature
      (B) LOCATION: 628..702
      (D) OTHER INFORMATION: /note- "Transmembrane domain"
   (xi) SEQUENCE DESCRIPTION: SEQ ID NO:5:
   ATG AGC TTT CCA TGT AAA TTT GTA GCC AGC TTC CTT CTG ATT TTC AAT    48
   Met Ser Phe Pro Cys Lys Phe Val Ala Ser Phe Leu Leu Ile Phe Asn
   -24             -20                 -15                 -10
   GTT TCT TCC AAA GGT GCA GTC TCC AAA GAG ATT ACG AAT GCC TTG GAA    96
   Val Ser Ser Lys Gly Ala Val Ser Lys Glu Ile Thr Asn Ala Leu Glu
                -5                   1               5
   ACC TGG GGT GCC TTG GGT CAG GAC ATC AAC TTG GAC ATT CCT AGT TTT   144
   Thr Trp Gly Ala Leu Gly Gln Asp Ile Asn Leu Asp Ile Pro Ser Phe
       10                  15                  20
   CAA ATG AGT GAT GAT ATT GAC GAT ATA AAA TGG GAA AAA ACT TCA GAC   192
   Gln Met Ser Asp Asp Ile Asp Asp Ile Lys Trp Glu Lys Thr Ser Asp
   25                  30                  35                  40
   AAG AAA AAG ATT GCA CAA TTC AGA AAA GAG AAA GAG ACT TTC AAG GAA   240
   Lys Lys Lys Ile Ala Gln Phe Arg Lys Glu Lys Glu Thr Phe Lys Glu
                   45                  50                  55
   AAA GAT ACA TAT AAG CTA TTT AAA AAT GGA ACT CTG AAA ATT AAG CAT   288
   Lys Asp Thr Tyr Lys Leu Phe Lys Asn Gly Thr Leu Lys Ile Lys His
               60                  65                  70
   CTG AAG ACC GAT GAT CAG GAT ATC TAC AAG GTA TCA ATA TAT GAT ACA   336
   Leu Lys Thr Asp Asp Gln Asp Ile Tyr Lys Val Ser Ile Tyr Asp Thr
           75                  80                  85
   AAA GGA AAA AAT GTG TTG GAA AAA ATA TTT GAT TTG AAG ATT CAA GAG   384
   Lys Gly Lys Asn Val Leu Glu Lys Ile Phe Asp Leu Lys Ile Gln Glu
       90                  95                 100
   AGG GTC TCA AAA CCA AAG ATC TCC TGG ACT TGT ATC AAC ACA ACC CTG   432
   Arg Val Ser Lys Pro Lys Ile Ser Trp Thr Cys Ile Asn Thr Thr Leu
   105                 110                 115                 120
   ACC TGT GAG GTA ATG AAT GGA ACT GAC CCC GAA TTA AAC CTG TAT CAA   480
   Thr Cys Glu Val Met Asn Gly Thr Asp Pro Glu Leu Asn Leu Tyr Gln
                   125                 130                 135
   GAT GGG AAA CAT CTA AAA CTT TCT CAG AGG GTC ATC ACA CAC AAG TGG   528
   Asp Gly Lys His Leu Lys Leu Ser Gln Arg Val Ile Thr His Lys Trp
               140                 145                 150
   ACC ACC AGC CTG AGT GCA AAA TTC AAG TGC ACA GCA GGG AAC AAA GTC   576
   Thr Thr Ser Leu Ser Ala Lys Phe Lys Cys Thr Ala Gly Asn Lys Val
           155                 160                 165
   AGC AAG GAA TCC AGT GTC GAG CCT GTC AGC TGT CCA GAG AAA GGT CTG   624
   Ser Lys Glu Ser Ser Val Glu Pro Val Ser Cys Pro Glu Lys Gly Leu
       170                 175                 180
   GAC ATC TAT CTC ATC ATT GGC ATA TGT GGA GGA GGC AGC CTC TTG ATG      672
   Asp Ile Tyr Leu Ile Ile Gly Ile Cys Gly Gly Gly Ser Leu Leu Met
   185                 190                 195                 200
   GTC TTT GTG GCA CTG CTC GTT TTC TAT ATC ACC AAA AGG AAA AAA CAG      720
   Val Phe Val Ala Leu Leu Val Phe Tyr Ile Thr Lys Arg Lys Lys Gln
                   205                 210                 215
   AGG AGT CGG AGA AAT GAT GAG GAG CTG GAG ACA AGA GCC CAC AGA GTA      768
   Arg Ser Arg Arg Asn Asp Glu Glu Leu Glu Thr Arg Ala His Arg Val
               220                 225                 230
   GCT ACT GAA GAA AGG GGC CGG AAG CCC CAC CAA ATT CCA GCT TCA ACC      816
   Ala Thr Glu Glu Arg Gly Arg Lys Pro His Gln Ile Pro Ala Ser Thr
           235                 240                 245
   CCT CAG AAT CCA GCA ACT TCC CAA CAT CCT CCT CCA CCA CCT GGT CAT      864
   Pro Gln Asn Pro Ala Thr Ser Gln His Pro Pro Pro Pro Pro Gly His
       250                 255                 260
   CGT TCC CAG GCA CCT AGT CAT CGT CCC CCG CCT CCT GGA CAC CGT GTT      912
   Arg Ser Gln Ala Pro Ser His Arg Pro Pro Pro Pro Gly His Arg Val
   265                 270                 275                 280
   CAG CAC CAG CCT CAG AAG AGG CCT CCT GCT CCG TCG GGC ACA CAA GTT      960
   Gln His Gln Pro Gln Lys Arg Pro Pro Ala Pro Ser Gly Thr Gln Val
                   285                 290                 295
   CAC CAG CAG AAA GGC CCG CCC CTC CCC AGA CCT CGA GTT CAG CCA AAA     1008
   His Gln Gln Lys Gly Pro Pro Leu Pro Arg Pro Arg Val Gln Pro Lys
               300                 305                 310
   CCT CCC CAT GGG GCA GCA GAA AAC TCA TTG TCC CCT TCC TCT AAT         1053
   Pro Pro His Gly Ala Ala Glu Asn Ser Leu Ser Pro Ser Ser Asn
           315                 320                 325
   TAA                                                                 1056
(2) INFORMATION FOR SEQ ID NO:6:
   (i) SEQUENCE CHARACTERISTICS:
      (A) LENGTH: 351 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Ser Phe Pro Cys Lys Phe Val Ala Ser Phe Leu Leu Ile Phe Asn
   -24             -20                 -15                 -10
   Val Ser Ser Lys Gly Ala Val Ser Lys Glu Ile Thr Asn Ala Leu Glu
               -5                  1               5
   Thr Trp Gly Ala Leu Gly Gln Asp Ile Asn Leu Asp Ile Pro Ser Phe
       10                  15                  20
   Gln Met Ser Asp Asp Ile Asp Asp Ile Lys Trp Glu Lys Thr Ser Asp
   25                  30                  35                  40
   Lys Lys Lys Ile Ala Gln Phe Arg Lys Glu Lys Glu Thr Phe Lys Glu
                   45                  50                  55
   Lys Asp Thr Tyr Lys Leu Phe Lys Asn Gly Thr Leu Lys Ile Lys His
               60                  65                  70
   Leu Lys Thr Asp Asp Gln Asp Ile Tyr Lys Val Ser Ile Tyr Asp Thr
           75                  80                  85
   Lys Gly Lys Asn Val Leu Glu Lys Ile Phe Asp Leu Lys Ile Gln Glu
       90                  95                  100
   Arg Val Ser Lys Pro Lys Ile Ser Trp Thr Cys Ile Asn Thr Thr Leu
   105                 110                 115                 120
   Thr Cys Glu Val Met Asn Gly Thr Asp Pro Glu Leu Asn Leu Tyr Gln
                   125                 130                 135
   Asp Gly Lys His Leu Lys Leu Ser Gln Arg Val Ile Thr His Lys Trp
               140                 145                 150
   Thr Thr Ser Leu Ser Ala Lys Phe Lys Cys Thr Ala Gly Asn Lys Val
           155                 160                 165
   Ser Lys Glu Ser Ser Val Glu Pro Val Ser Cys Pro Glu Lys Gly Leu
       170                 175                 180
   Asp Ile Tyr Leu Ile Ile Gly Ile Cys Gly Gly Gly Ser Leu Leu Met
   185                 190                 195                 200
   Val Phe Val Ala Leu Leu Val Phe Tyr Ile Thr Lys Arg Lys Lys Gln
                   205                 210                 215
   Arg Ser Arg Arg Asn Asp Glu Glu Leu Glu Thr Arg Ala His Arg Val
               220                 225                 230
   Ala Thr Glu Glu Arg Gly Arg Lys Pro His Gln Ile Pro Ala Ser Thr
           235                 240                 245
   Pro Gln Asn Pro Ala Thr Ser Gln His Pro Pro Pro Pro Pro Gly His
       250                 255                 260
   Arg Ser Gln Ala Pro Ser His Arg Pro Pro Pro Pro Gly His Arg Val
   265                 270                 275                 280
   Gln His Gln Pro Gln Lys Arg Pro Pro Ala Pro Ser Gly Thr Gln Val
                   285                 290                 295
   His Gln Gln Lys Gly Pro Pro Leu Pro Arg Pro Arg Val Gln Pro Lys
   300 305 310
   Pro Pro His Gly Ala Ala Glu Asn Ser Leu Ser Pro Ser Ser Asn
   315 320 325
(2) INFORMATION FOR SEQ ID NO:7:
   (i) SEQUENCE CHARACTERISTICS:
      (A) LENGTH: 1050 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (ix) FEATURE:
      (A) NAME/KEY: CDS
      (B) LOCATION: 1..1041
   (ix) FEATURE:
      (A) NAME/KEY: sig_peptide
      (B) LOCATION: 1..84
   (ix) FEATURE:
      (A) NAME/KEY: mat_peptide
      (B) LOCATION: 85..1041
   (ix) FEATURE:
      (A) NAME/KEY: misc_feature
      (B) LOCATION: 85..1041
      (D) OTHER INFORMATION: /note- "LFA3TIP"
   (ix) FEATURE:
      (A) NAME/KEY: misc_feature
      (B) LOCATION: 360..361
      (D) OTHER INFORMATION: /note- "LFA-3/IgG fusion point"
   (xi) SEQUENCE DESCRIPTION: SEQ ID NO:7:
   ATG GTT GCT GGG AGC GAC GCG GGG CGG GCC CTG GGG GTC CTC AGC GTG     48
   Met Val Ala Gly Ser Asp Ala Gly Arg Ala Leu Gly Val Leu Ser Val
   -28         -25                 -20                 -15
   GTC TGC CTG CTG CAC TGC TTT GGT TTC ATC AGC TGT TTT TCC CAA CAA     96
   Val Cys Leu Leu His Cys Phe Gly Phe Ile Ser Cys Phe Ser Gln Gln
           -10                 -5                  1
   ATA TAT GGT GTT GTG TAT GGG AAT GTA ACT TTC CAT GTA CCA AGC AAT    144
   Ile Tyr Gly Val Val Tyr Gly Asn Val Thr Phe His Val Pro Ser Asn
    5                  10                  15                  20
   GTG CCT TTA AAA GAG GTC CTA TGG AAA AAA CAA AAG GAT AAA GTT GCA        192
   Val Pro Leu Lys Glu Val Leu Trp Lys Lys Gln Lys Asp Lys Val Ala
                    25                  30                  35
   GAA CTG GAA AAT TCT GAA TTC AGA GCT TTC TCA TCT TTT AAA AAT AGG        240
   Glu Leu Glu Asn Ser Glu Phe Arg Ala Phe Ser Ser Phe Lys Asn Arg
                40                  45                  50
   GTT TAT TTA GAC ACT GTG TCA GGT AGC CTC ACT ATC TAC AAC TTA ACA        288
   Val Tyr Leu Asp Thr Val Ser Gly Ser Leu Thr Ile Tyr Asn Leu Thr
            55                  60                  65
   TCA TCA GAT GAA GAT GAG TAT GAA ATG GAA TCG CCA AAT ATT ACT GAT        336
   Ser Ser Asp Glu Asp Glu Tyr Glu Met Glu Ser Pro Asn Ile Thr Asp
        70                  75                  80
   ACC ATG AAG TTC TTT CTT TAT GTC GAC AAA ACT CAC ACA TGC CCA CCG        384
   Thr Met Lys Phe Phe Leu Tyr Val Asp Lys Thr His Thr Cys Pro Pro
    85                  90                  95                 100
   TGC CCA GCA CCT GAA CTC CTG GGG GGA CCG TCA GTC TTC CTC TTC CCC        432
   Cys Pro Ala Pro Glu Leu Leu Gly Gly Pro Ser Val Phe Leu Phe Pro
                   105                 110                 115
   CCA AAA CCC AAG GAC ACC CTC ATG ATC TCC CGG ACC CCT GAG GTC ACA        480
   Pro Lys Pro Lys Asp Thr Leu Met Ile Ser Arg Thr Pro Glu Val Thr
               120                 125                 130
   TGC GTG GTG GTG GAC GTG AGC CAC GAA GAC CCT GAG GTC AAG TTC AAC        528
   Cys Val Val Val Asp Val Ser His Glu Asp Pro Glu Val Lys Phe Asn
           135                 140                 145
   TGG TAC GTG GAC GGC GTG GAG GTG CAT AAT GCC AAG ACA AAG CCG CGG        576
   Trp Tyr Val Asp Gly Val Glu Val His Asn Ala Lys Thr Lys Pro Arg
       150                 155                 160
   GAG GAG CAG TAC AAC AGC ACG TAC CGG GTG GTC AGC GTC CTC ACC GTC        624
   Glu Glu Gln Tyr Asn Ser Thr Tyr Arg Val Val Ser Val Leu Thr Val
   165                 170                 175                 180
   CTG CAC CAG GAC TGG CTG AAT GGC AAG GAG TAC AAG TGC AAG GTC TCC        672
   Leu His Gln Asp Trp Leu Asn Gly Lys Glu Tyr Lys Cys Lys Val Ser
                   185                 190                 195
   AAC AAA GCC CTC CCA GCC CCC ATC GAG AAA ACC ATC TCC AAA GCC AAA        720
   Asn Lys Ala Leu Pro Ala Pro Ile Glu Lys Thr Ile Ser Lys Ala Lys
               200                 205                 210
   GGG CAG CCC CGA GAA CCA CAG GTG TAC ACC CTG CCC CCA TCC CGG GAT        768
   Gly Gln Pro Arg Glu Pro Gln Val Tyr Thr Leu Pro Pro Ser Arg Asp
           215                 220                 225
   GAG CTG ACC AAG AAC CAG GTC AGC CTG ACC TGC CTG GTC AAA GGC TTC        816
   Glu Leu Thr Lys Asn Gln Val Ser Leu Thr Cys Leu Val Lys Gly Phe
       230                 235                 240
   TAT CCC AGC GAC ATC GCC GTG GAG TGG GAG AGC AAT GGG CAG CCG GAG        864
   Tyr Pro Ser Asp Ile Ala Val Glu Trp Glu Ser Asn Gly Gln Pro Glu
   245                 250                 255                 260
   AAC AAC TAC AAG ACC ACG CCT CCC GTG CTG GAC TCC GAC GGC TCC TTC        912
   Asn Asn Tyr Lys Thr Thr Pro Pro Val Leu Asp Ser Asp Gly Ser Phe
                   265                 270                 275
   TTC CTC TAC AGC AAG CTC ACC GTG GAC AAG AGC AGG TGG CAG CAG GGG        960
   Phe Leu Tyr Ser Lys Leu Thr Val Asp Lys Ser Arg Trp Gln Gln Gly
               280                 285                 290
   AAC GTC TTC TCA TGC TCC GTG ATG CAT GAG GCT CTG CAC AAC CAC TAC       1008
   Asn Val Phe Ser Cys Ser Val Met His Glu Ala Leu His Asn His Tyr
           295                 300                 305
   ACG CAG AAG AGC CTC TCC CTG TCT CCG GGT AAA TGAGTGCGG                 1050
   Thr Gln Lys Ser Leu Ser Leu Ser Pro Gly Lys
       310                 315
(2) INFORMATION FOR SEQ ID NO:8:
   (i) SEQUENCE CHARACTERISTICS:
      (A) LENGTH: 347 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                  -5                   1
   Ile Tyr Gly Val Val Tyr Gly Asn Val Thr Phe His Val Pro Ser Asn
     5                  10                  15                  20
   Val Pro Leu Lys Glu Val Leu Trp Lys Lys Gln Lys Asp Lys Val Ala
                    25                  30                  35
   Glu Leu Glu Asn Ser Glu Phe Arg Ala Phe Ser Ser Phe Lys Asn Arg
                40                  45                  50
   Val Tyr Leu Asp Thr Val Ser Gly Ser Leu Thr Ile Tyr Asn Leu Thr
            55                  60                  65
   Ser Ser Asp Glu Asp Glu Tyr Glu Met Glu Ser Pro Asn Ile Thr Asp
        70                  75                  80
   Thr Met Lys Phe Phe Leu Tyr Val Asp Lys Thr His Thr Cys Pro Pro
    85                  90                  95                 100
   Cys Pro Ala Pro Glu Leu Leu Gly Gly Pro Ser Val Phe Leu Phe Pro
                   105                 110                 115
   Pro Lys Pro Lys Asp Thr Leu Met Ile Ser Arg Thr Pro Glu Val Thr
               120                 125                 130
   Cys Val Val Val Asp Val Ser His Glu Asp Pro Glu Val Lys Phe Asn
           135                 140                 145
   Trp Tyr Val Asp Gly Val Glu Val His Asn Ala Lys Thr Lys Pro Arg
       150                 155                 160
   Glu Glu Gln Tyr Asn Ser Thr Tyr Arg Val Val Ser Val Leu Thr Val
   165                 170                 175                 180
   Leu His Gln Asp Trp Leu Asn Gly Lys Glu Tyr Lys Cys Lys Val Ser
                   185                 190                 195
   Asn Lys Ala Leu Pro Ala Pro Ile Glu Lys Thr Ile Ser Lys Ala Lys
               200                 205                 210
   Gly Gln Pro Arg Glu Pro Gln Val Tyr Thr Leu Pro Pro Ser Arg Asp
           215                 220                 225
   Glu Leu Thr Lys Asn Gln Val Ser Leu Thr Cys Leu Val Lys Gly Phe
       230                 235                 240
   Tyr Pro Ser Asp Ile Ala Val Glu Trp Glu Ser Asn Gly Gln Pro Glu
   245                 250                 255                 260
   Asn Asn Tyr Lys Thr Thr Pro Pro Val Leu Asp Ser Asp Gly Ser Phe
                   265                 270                 275
   Phe Leu Tyr Ser Lys Leu Thr Val Asp Lys Ser Arg Trp Gln Gln Gly
               280                 285                 290
   Asn Val Phe Ser Cys Ser Val Met His Glu Ala Leu His Asn His Tyr
           295                 300                 305
   Thr Gln Lys Ser Leu Ser Leu Ser Pro Gly Lys
       310                 315